# EUROPÄISCHE PATENTSCHRIFT

(11) **EP 2 735 605 B1**
(45) Veröffentlichungstag und Bekanntmachung des Hinweises auf die Patenterteilung: **13.06.2018**
(21) Anmeldenummer: 13190595.2
(22) Anmeldetag: 29.10.2013
(51) Int. Cl.: C11B 1/10

(54) **Verfahren zur Isolierung von Rhamnolipiden**
Process for the isolation of rhamnolipids
Procédé d'isolation de rhamnolipides

(30) Priorität: 26.11.2012 DE 102012221519
(43) Veröffentlichungstag der Anmeldung: 28.05.2014
(73) Patentinhaber: Evonik Degussa GmbH, 45128 Essen (DE)
(72) Erfinder: Schilling, Martin, 53121 Bonn (DE); Ruetering, Marius, 59872 Meschede (DE); Dahl, Verena, 50858 Köln (DE); Cabirol, Fabien, 45133 Essen (DE)

(56) Entgegenhaltungen:
- WO-A2-93/14767
- US-A- 5 466 675
- ABDEL-MOUWGOUD, A. M., ET AL.: "CHARACTERIZATION OF RHAMNOLIPID PRODUCED BY PSEUDOMONAS AERUGINOSA ISOLATE BS20", APPLIED BIOCHEMISTRY AND BIOTECHNOLOGY, Bd. 157, 2009, Seiten 329-345, XP002720802, Humana Press, Inc. ISSN: 0273-2289
- ALEJANDRO P. ROONEY ET AL: "Isolation and characterization of rhamnolipid-producing bacterial strains from a biodiesel facility", FEMS MICROBIOLOGY LETTERS, Bd. 295, Nr. 1, 1. Juni 2009 (2009-06-01), Seiten 82-87, XP055103888, ISSN: 0378-1097, DOI: 10.1111/j.1574-6968.2009.01581.x
- NEISSA M PINZON ET AL: "Analysis of rhamnolipid biosurfactants by methylene blue complexation", APPLIED MICROBIOLOGY AND BIOTECHNOLOGY, SPRINGER, BERLIN, DE, vol. 82, no. 5, 13 February 2009 (2009-02-13), pages 975-981, XP019705540, ISSN: 1432-0614

## Beschreibung

### Gebiet der Erfindung

Gegenstand der Erfindung ist ein Verfahren zur Isolierung von Rhamnolipiden.

### Stand der Technik

Rhamnolipide sind grenzflächenaktive Glykolipide und Stoffwechselprodukte bestimmter Mikroorganismen, von denen der bekannteste Produzent wohl *Pseudomonas aeruginosa* darstellt.

Rhamnolipide verfügen über besondere tensidische Eigenschaften wie beispielsweise ein starkes Schaumvermögen und sind für verschiedenste technische Anwendungen interessant.
Die Tatsache, dass sie unter milden Bedingungen mittels Fermentation basierend auf nachwachsenden Rohstoffen herstellbar sind und damit gut zu den zeitgenössischen Megatrends "Nachhaltigkeit" und "grünen Chemie" passen, hat das Interesse an den Rhamnolipiden in den letzten Jahren stetig zunehmen lassen.
Rhamnolipide sind allerdings bisher nur in kleinen Mengen und hohen Preisen auf dem Markt verfügbar und können nur in Nischenanwendungen mit den typischerweise in zum Beispiel Köperpflegeprodukten und Haushaltsreinigern angewandten chemisch-synthetischen Tensiden konkurrieren. Ein Grund für die hohen Kosten liegt insbesondere in der schwierigen Aufarbeitung des Fermentationsproduktes von den Fermentationsbrühen, welche eine große Anzahl an verunreinigenden Nebenkomponenten enthalten. Hierzu gehören unvollständig umgesetzte, lipophile und amphiphile Substrate (z.B. Tri- und Paritalglyceride sowie Fettsäuren), lipophile und amphiphile Zellbestandteile (z.B. Phospholipide der Zellmembranen), sowie Antischaummittel (z.B. Silikon-, Pflanzenöl- oder Polyether-basiert), die zur Schaumkontrolle während der Fermentation eingesetzt werden.
Insbesondere die Antischaummittel stören in der späteren Anwendung und sind aufgrund der amphiphilen Natur der Rhamnolipide nur schwer von diesen zu trennen. Neben den schauminhibierenden Komponenten müssen in vielen Fällen auch hydrophile Fermentationsnebenprodukte, wie z.B. Proteine und mikrobielle Polysaccharide abgetrennt werden, da diese aus anderen Gründen im Endprodukt stören können (mikrobielle Stabilität, toxikologische Eigenschaften etc.). Zu guter Letzt ist bei der Aufarbeitung auch eine starke Aufkonzentrierung der wässrigen RL Lösung zu erreichen, da Tensidlösungen auf Marktseite in möglichst hochkonzentrierter Form gefordert werden. Aus ökologischen Gründen sollen möglichst geringe Mengen Wasser mit dem Produkt transportiert werden, um einen möglichst geringen Treibstoffverbrauch pro kg Aktivsubstanz zu gewährleisten. Die durch Fermentation erreichbaren Produktkonzentrationen sind relativ niedrig, aus der Literatur sind bisher keine Konzentrationen größer 100 g/l bekannt.

Aus der Literatur ist eine Reihe von Methoden bekannt, um Rhamnolipide, die häufig eine Mischung verschiedener Formen darstellen (z.B. mono- und di-Rhamnolipide), aufzureinigen und von den vorgenannten Verunreinigungen zu trennen. Verschiedene, verfahrenstechnisch anspruchsvolle Methoden, wie z.B. der Einsatz von Membranfiltration, die Adsorption an Ionenaustauscher sowie die Schaumfraktionierung während der Fermentation erlauben eine Teilaufreinigung, allerdings können durch einen einzigen dieser Verfahrensschritte weder die hydrophilen, noch die hydrophobe Verunreinigungen in ausreichendem Maß abgetrennt werden. Zudem ist ein kosteneffizienter Einsatz in industriellem Maßstab für die Aufreinigung von RLs nicht untersucht und tendenziell zu teuer um bezüglich der Herstellkosten mit den klassisch chemisch-synthetischen Tensiden konkurrenzfähig zu sein. Dies gilt natürlich auch für die verfügbaren chromatographischen Verfahren. Sarachat et al. (2010) beschreiben die saure Extraktion von Fermentationsbrühen mit einem organischen Lösungsmittel beziehungsweise Lösungsmittelgemisch mittlerer Polarität, wie z.B. Ethylacetat, Chloroform-Methanol, Chloroform-Ethanol oder Dichlormethan, bei der zunächst lipophile Komponenten mit extrahiert werden. Die Rhamnolipide werden in einem zweiten Schritt durch schrittweise Hinzugabe eines unpolareren Lösungsmittels, wie z.B. Hexan oder Chloroform unter Kühlung zur Fällung gebracht und weiter gereinigt.
Ein offensichtlicher Nachteil dieses Verfahrens ist, dass die Zugabe von toxikologisch bedenklichen, erdölbasierten unpolaren Lösungsmitteln wie Hexan oder Chloroform und ein Kristallisationsschritt mit Kühlung erforderlich sind. Neben den ebenfalls höheren Kosten, den verfahrenstechnischen und sicherheitstechnischen Anforderungen ist ein solches Verfahren auch im Sinne einer nachhaltigen, grünen Chemie nicht ideal. Zudem existieren bereits jetzt regulatorische Anforderungen, die die Öko-Zertifizierung und -vermarktung eines aus einem solchen Prozess stammenden Produktes nicht erlauben.

Mawgoud et al. beschreiben in Appl Biochem Biotechnol. 2009 May:157(2):329-45 die Charakterisierung von Rhamnolipiden des Stammes *Pseudomonas aeruginosa* Bs20. Rooney et a. beschreiben in FEMS Microbiol Lett. 2009 Jun;295(1):82-7 die Isolierung und Charakterisierung eines Rhamnolipid-Produzenten von einer Biodieselanlage. Pinzon et al. beschreiben in Appl Microbiol Biotechnol. 2009 Apr;82(5):975-81 die Analyse von Rhamnolipiden mit Hilfe von Methylen-Blau-Komplexierung.

Aufgabe der Erfindung war es, ein Verfahren zur Aufreinigung von Rhamnolipiden zur Verfügung zu stellen, was mindestens einen der Nachteile des Standes der Technik zu überwinden vermag.

### Beschreibung der Erfindung

Überraschenderweise wurde gefunden, dass das im Folgenden beschriebene Verfahren die der Erfindung gestellte Aufgabe zu lösen vermag.

Gegenstand der vorliegenden Erfindung ist daher ein Verfahren zur Isolierung von Rhamnolipiden wie in den vorliegenden Ansprüchen beansprucht.

Vorteil des erfindungsgemäßen Verfahrens ist es, dass wenig Lösungsmittel gebraucht wird.
Ein weiterer Vorteil ist es, dass die eingesetzten Lösungsmittel toxikologisch und sicherheitstechnisch unbedenklich sind.
Noch ein weiterer Vorteil ist es, dass Lösungsmittel aus nachhaltigen Quellen stammend eingesetzt werden können.
Noch ein Vorteil ist es, dass das erfindungsgemäße Verfahren bei energetisch günstigen Temperaturen durchgeführt werden kann.
Noch ein Vorteil ist es, dass das erfindungsgemäße Verfahren mit einfachen technischen Geräten durchgeführt werden kann.
Ein weiterer Vorteil des erfindungsgemäßen Verfahrens ist es, dass es die Aufreinigung/Aufkonzentrierung großer Produktmengen in kürzester Zeit ermöglicht. Noch ein Vorteil ist es, dass in dem erfindungsgemäßen Verfahren das aufkonzentrierte und aufgereinigte Produkt nicht als Feststoff, sondern als flüssige Phase erhalten wird, was die verfahrenstechnische Weiterverarbeitung erleichtert. Die anfallende, hochkonzentrierte Lösung stellt das fertige Endprodukt dar.
Ein weiterer Vorteil des erfindungsgemäßen Verfahrens ist es, dass es zur Aufarbeitung sowohl von Fermentationsbrühen als auch von bereits teilaufgereinigten Rhamnolipiden geeignet ist.

Das erfindungsgemäße Verfahren zur Isolierung von Rhamnolipiden umfasst die Verfahrensschritte
A) Bereitstellen eines mindestens ein Rhamnolipid enthaltenden wässrigen Mediums mit einem pH-Wert kleiner 6, insbesondere kleiner 5,
B) in Kontakt Bringen des Mediums mit mindestens einem organischen Lösungsmittel unter Erhalt eines Mehrphasensystems und Abtrennen der wässrigen Phase,
C) Erhöhung des pH-Wertes auf einen pH-Wert von 6 oder größer unter Erhalt eines mehrphasigen organischen Systems,
D) Abtrennen einer mit Rhamnolipid angereicherten organischen Phase und
E) gegebenenfalls weitere Aufreinigung des Rhamnolipides. und ist dadurch gekennzeichnet, das gesamte wässrige Medium in Verfahrensschritt A) zwischen 10 g/L und 300 g/L, insbesondere zwischen 50 g/L und 150 g/L Rhamnolipide enthält, wobei sich die Literangabe auf das gesamte wässrige Medium beziehen.

Unter dem Begriff "Rhamnolipid" im Zusammenhang mit der vorliegenden Erfindung werden insbesondere Verbindungen der allgemeinen Formel (I) oder dessen Salze verstanden, 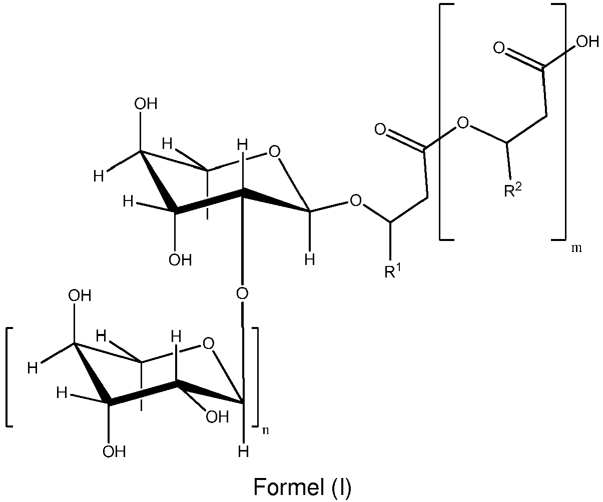 wobei
m = 2, 1 oder 0, insbesondere 1 oder 0,
n = 1 oder 0, insbesondere 1,
R¹ und R² = unabhängig voneinander gleicher oder verschiedener organischer Rest mit 2 bis 24, bevorzugt 5 bis 13 Kohlenstoffatomen, insbesondere gegebenenfalls verzweigter, gegebenenfalls substituierter, insbesondere hydroxy-substituierter, gegebenenfalls ungesättigter, insbesondere gegebenenfalls einfach, zweifach oder dreifach ungesättigter, Alkylrest, bevorzugt solcher ausgewählt aus der Gruppe bestehend aus Pentenyl, Heptenyl, Nonenyl, Undekenyl und Tridekenyl und (CH₂)ₒ-CH₃ mit o = 1 bis 23, bevorzugt 4 bis 12,
Der "pH-Wert" im Zusammenhang mit der vorliegenden Erfindung ist definiert als der Wert, welcher für entsprechenden Stoff bei 25 °C nach fünf Minuten Rühren mit einer gemäß ISO 4319 (1977) kalibrierten pH-Elektrode gemessen wird.
Unter dem Begriff "wässriges Medium" im Zusammenhang mit der vorliegenden Erfindung ist eine Zusammensetzung zu verstehen, die mindestens 5 Gew.-% Wasser, bezogen auf die betrachtete Gesamtzusammensetzung enthält.
Alle angegebenen Prozent (%) sind, wenn nicht anders angegeben, Massenprozent.

Insbesondere bevorzugt ist in dem erfindungsgemäßen Verfahren das wässrige Medium in Verfahrensschritt A) eine zellfreie oder zellhaltige Fermentationsbrühe, bevorzugt eine zellfreie.

Insbesondere ist das erfindungsgemäße Verfahren dadurch gekennzeichnet, dass das wässrige Medium in Verfahrensschritt A) einen pH-Wert von 2 bis 4,5 insbesondere von 3,5 bis 4 aufweist.
Der pH-Wert kann in diesem Zusammenhang vorteilhaft mit einer Säure, insbesondere einer anorganischen Säure, die besonders bevorzugt ausgewählt aus HCl, H₂SO₄, Salpetersäure, Phosphorsäure und Kohlensäure, insbesondere HCl ist, eingestellt werden.

Es ist vorteilhaft für das erfindungsgemäße Verfahren, wenn nach Verfahrensschritt D) in der mit Rhamnolipid angereicherten organischen Phase eine bestimmte Menge Wasser enthalten ist; dies hat den technischen Effekt, dass man durch einfaches Entfernen, beispielsweise Abdestillieren, des organischen Lösungsmittels eine hochkonzentrierte, wässrige Rhamnolipidfraktion erhalten kann; deswegen ist ein bevorzugtes erfindungsgemäßes Verfahren dadurch gekennzeichnet, dass das organische Lösungsmittel oder die Mischung der organischen Lösungsmittel des Verfahrensschrittes B) Wasser in einer Menge von 0,1 bis 30 Gew.-%, bevorzugt 0,5 bis 7 Gew.-%, zu lösen vermag, wobei sich die Gewichtsprozente auf die Summe von organischem Lösungsmittel und Wasser beziehen.

Bevorzugte organische Lösungsmittel des Verfahrensschrittes B) sind ausgewählt aus Methylacetat, Ethylacetat, Propylacetat, Isopropylacetat, Butylacetat, Isoamylacetat, Butan-1-ol, Butan-2-ol und Diethylether, wobei Ethylacetat besonders bevorzugt ist. Es ist erfindungsgemäß bevorzugt, wenn in dem erfindungsgemäßen Verfahren das Volumenverhältnis bei 20 °C von wässrigem Medium zu organischem Lösungsmittel oder zu der Mischung der organischen Lösungsmittel vor in Kontakt Bringen von 0,2:1 bis 5:1, bevorzugt von 2:1 bis 4:1 und besonders bevorzugt von 2,9:1 bis 3,1:1 beträgt.

Eine bevorzugte Ausführungsform des erfindungsgemäßen Verfahrens ist dadurch gekennzeichnet, dass in Verfahrensschritt C) der pH-Wert auf von 6,1 bis 9, bevorzugt auf von 6,5 bis 8, besonders bevorzugt auf von 6,9 bis 7,1, erhöht wird.

Es ist erfindungsgemäß bevorzugt, wenn in dem erfindungsgemäßen Verfahren in Verfahrensschritt C) der pH-Wert durch eine anorganische Base, insbesondere Erdalkali-, Alkalibase, bevorzugt wässrige, oder Ammoniak erhöht wird, wobei NaOH und KOH besonders bevorzugt sind.

Ein Vorteil des erfindungsgemäßen Verfahrens ist es, dass es zum Großteil bei Umgebungstemperatur durchgeführt werden kann, somit werden die Verfahrensschritte B), C) und D) bevorzugt in einem Temperaturbereich von 10 bis 50 °C, bevorzugt 15 bis 30, besonders bevorzugt 18 bis 25 durchgeführt.

In Verfahrensschritt E) kann das Rhamnolipid bei Bedarf weiter aufgereinigt werden. Eine bevorzugte Ausführungsform des erfindungsgemäßen Verfahrens ist dadurch gekennzeichnet, dass in Verfahrensschritte E) vorhandenes organisches Lösungsmittel durch mindestens teilweises Verdampfen von dem Rhamnolipid abgetrennt wird. In diesem Zusammenhang ist es bevorzugt, dass das Verdampfen des organischen Lösungsmittels unter vermindertem Druck und erhöhter Temperatur durchgeführt wird. Insbesondere ein Druckbereich von 0,01 bis 1 bar, bevorzugt von 0,15 bis 0,3 bar in Verbindung mit einer Temperatur von 20 bis 80 °C, bevorzugt von 40 bis 60 °C, hat sich als vorteilhaft herausgestellt.

Das erfindungsgemäße Verfahren kann einen weiteren Verfahrensschritt F) umfassen, der die Rückgewinnung von gegebenenfalls enthaltenem Antischaum-Mittel, insbesondere aus den organischen Phasen, beinhaltet. Dieses kann dann vorteilhaft in einer nächsten Fermentation wiederverwertet werden.

In den nachfolgend aufgeführten Beispielen wird die vorliegende Erfindung beispielhaft beschrieben, ohne dass die Erfindung, deren Anwendungsbreite sich aus der gesamten Beschreibung und den Ansprüchen ergibt, auf die in den Beispielen genannten Ausführungsformen beschränkt sein soll.

Der Umfang der Erfindung ist durch die Ansprüche definiert.

### Beispiele:

### Beispiel 1: Abtrennung und Aufkonzentrierung von Rhamnolipiden aus einem Rhamnolipid/Fettsäure-Gemisch mittels Extraktion und pH induzierter Phasentrennung

Als Ausgangsprodukt für die nachfolgend beschriebene Aufreinigung und Aufkonzentrierung wurde ein fermentativ hergestelltes Rhamnolipid Rohprodukt verwendet. Dieses bestand zu etwa 50% aus verschiedenen Rhamnolipiden sowie zu circa 20% aus unterschiedlichen, fettsäureartiger Verunreinigungen, welche entweder durch das Fermentationssubstrat eingetragen wurden oder auch während der Fermentation als Nebenprodukt gebildet wurden. Hierbei handelt es sich hauptsächlich um C10 Hydroxyfettsäuren sowie deren Dimere und einfach ungesättigte C12 Fettsäuren. Zur Detektion der Verunreinigungen in allen Aufbereitungsschritten wurde unter anderem ein speziell hierfür entwickeltes dünnschichtchromatographisches Verfahren verwendet. Die Aufreinigung von Rhamnolipiden erfolgte ausgehend von einer wässrigen Lösung mit einer RL Konzentration von 50 g/L. Der Anteil der Fettsäuren in der wässrigen Lösung betrug 20 g/L.
In Fermentationsbrühen bzw. teilaufgereinigten Rohprodukten befinden sich häufig Proteine, die die Phasentrennung nach der Extraktion erschweren und zur Bildung großer Interphasen führen. Damit ist meist auch ein Produkt- bzw. Ausbeuteverlust verbunden. Um dies zu verhindern, wurden die Proteine zunächst durch Zugabe 1 Gew. % einer kommerziellen Proteasepräparation (Alcalase, Novozymes) bei einem pH = 7 und 60 °C hydrolysiert. Dadurch wurde die anschließende Extraktion stark vereinfacht. Zur Extraktion der Rhamnolipide wurde die wässrige Lösung mit Hilfe von konzentrierter HCl auf einen pH-Wert von 4,0 eingestellt. Anschließend wurde mit gleichem Volumen Ethylacetat extrahiert. Die Phasentrennung im Scheidetrichter nach erfolgreicher Extraktion erfolgte schnell und zuverlässig bei Raumtemperatur (25 °C). Hydrophile Verunreinigungen verblieben in diesem Schritt bereits in der wässrigen Phase. Die lipophileren Verunreinigungen und nahezu 100% der enthaltenen Rhamnolipide lösten sich in der organischen Phase.
Die rhamnolipidhaltige organische Phase wurde abgetrennt und weiter prozessiert. Durch Zugabe von 50%iger KOH (aq) wurde der pH-Wert der Lösung auf pH 7 eingestellt. Eine plötzlich eintretende Trübung der Lösung in diesem pH Bereich indizierte das Auftreten einer weiteren organischen Phase; die organischen Phasen wurden wiederum im Scheidetrichter schnell und unkompliziert bei Raumtemperatur (25 °C) getrennt, wobei sich die Rhamnolipide vornehmlich in der unteren Phase angereichert hatten und die lipophilen Verunreinigungen größtenteils in der oberen Phase verblieben.
Somit wurde nicht nur eine starke Aufkonzentrierung der Rhamnolipiden, sondern zugleich auch eine effektive Aufreinigung derselbigen erreicht. Nach Abtrennen der unteren, Rhamnolipide enthaltenden Phase wurde das organische Lösungsmittel mittels einfacher Destillation bei 0,2 bar und 60°C abgezogen.

Die erhaltene Rhamnolipid Fraktion wies eine Konzentration von 500 g/L auf.
Die Reinheit betrug 80%. Dies entspricht einer 10 fachen Aufkonzentrierung mit einem Aufreinigungfaktor von 1,6 bei insgesamt 95% Ausbeute.

Das fertige Endprodukt wies deutlich bessere anwendungstechnische Eigenschaften auf als die verunreinigte Ausganglösung auf. Insbesondere die Schaumeigenschaften wurden hierdurch verbessert. Dies wurde mit einem Schaumtester der Firma SITA (R-2000) überprüft. Das aufgereinigte Produkt zeigte eine deutlich schnellere Schaumbildungskinetik und das erreichte Schaumvolumen war ebenfalls deutlich höher.

### Beispiel 2: Abtrennung und Aufkonzentrierung von Rhamnolipiden aus einem Rhamnolipide/Antischaum Gemisch mittels Extraktion und pH induzierter Phasentrennung

Zur Abtrennung von Entschäumer von einer Rhamnolipid Lösung wurde ein kommerziell erhältliches Rhamnolipid Gemisch (JBR 505) von Jeneil Biosurfactants verwendet.

Die Aufreinigung von Rhamnolipiden erfolgte ausgehend von einer wässrigen Lösung mit einer Konzentration von 50 g/L. Der silikonhaltige DOW Corning 1500 Entschäumer wurde in einer Endkonzentration von 20 g/L zugesetzt.
Zur Extraktion der Rhamnolipide wurde die wässrige Lösung mit Hilfe von konzentrierter HCl auf einen pH-Wert von 4,0 eingestellt. Anschließend wurde mit gleichem Volumen Ethylacetat extrahiert. Die Phasentrennung im Scheidetrichter nach erfolgreicher Extraktion erfolgte schnell und zuverlässig bei Raumtemperatur (25 °C). Hydrophile Verunreinigungen verblieben in diesem Schritt bereits in der wässrigen Phase. Die lipophileren Verunreinigungen und nahezu 100% der enthaltenen Rhamnolipide lösten sich in der organischen Phase.
Die rhamnolipidhaltige organische Phase wurde abgetrennt und weiter prozessiert. Durch Zugabe von 50%iger KOH (aq) wurde der pH-Wert der Lösung auf pH 7 eingestellt. Eine plötzlich eintretende Trübung der Lösung in diesem pH Bereich indizierte das Auftreten einer weiteren organischen Phase; die organischen Phasen wurden wiederum im Scheidetrichter schnell und unkompliziert bei Raumtemperatur (25 °C) getrennt, wobei sich die Rhamnolipide vornehmlich in der unteren Phase angereichert hatten und die Mehrheit der lipophilen Verunreinigungen sowie nahezu 100% des Entschäumers in der oberen Phase verblieben.
Somit wurde neben der Aufreinigung und starken Aufkonzentrierung der Rhamnolipiden zugleich auch eine effektive Abtrennung vom Entschäumer erreicht. Die Abtrennung des Entschäumers wurde mittels quantitativer ¹H-NMR Analytik nachgewiesen. Das für den Polydimethylsiloxanentschäumer typische Signal bei einer chemischen Verschiebung von etwa - 0.1 ppm konnte in der unteren rhamnolipidhaltigen Phase praktisch nicht mehr detektiert werden, dafür aber deutlich in der oberen Ethylacetatphase. Nach Abtrennen der unteren, Rhamnolipide enthaltenden Phase wurde das organische Lösungsmittel mittels einfacher Destillation bei 0,2 bar und 60°C abgezogen.

Die erhaltene Rhamnolipid Fraktion wies eine Konzentration von 450 g/L auf.
Die Reinheit betrug 75%. Dies entspricht einer 9 fachen Aufkonzentrierung mit einem Aufreinigungsfaktor von 1,5 bei insgesamt 90% Ausbeute.

Das fertige Endprodukt wies deutlich bessere anwendungstechnische Eigenschaften auf als die verunreinigte Ausganglösung auf.

### Beispiel 3: Abtrennung und Aufkonzentrierung von Rhamnolipiden aus einer Rhamnolipid und Antischaum enthaltenden Fermentationsbrühe

Eine Fermentation mit einem die Rhamnolipid-Biosynthesegene RHIA, RHIB und RhIC enthaltenden *Pseudomonas putida* Stamm wird durchgeführt. Die Vorkultur im Schüttelkolben wird wie in WO2012013554A1 beschrieben durchgeführt. Für die Hauptkultur kommt ebenfalls ein Mineralmedium (M9) zum Einsatz. Die Fermentation wird kohlenstofflimitiert über eine Glukosezufütterung in einem 2 Liter Fermenter geführt. Die Glukosezufütterung erfolgt anhand des Gelöstsauerstoffsignals. Der Gelöstsauerstoff wird bei 20 % Sättigung über die Rührerdrehzahl reguliert. Der pH Wert wird über eine pH Elektrode und Zugabe von NH₄SO₄ auf 7 reguliert. Um das Überschäumen der Fermentationsbrühe zu verhindern wird in einem Fall der silikonhaltige Entschäumer DOW Corning 1500, im anderen Fall ein Sonnenblumenöl zudosiert. Die Fermentation wird über 4 Tage bis zu einer Biotrockenmasse von 15 g/l geführt. Die Rhamnolipidkonzentration wird über HPLC ermittelt und beträgt 9,8 g/l. Die am Ende der Fermentation erhaltene Brühe zeigt weder im Falle des eingesetzten DOW Corning 1500 Entschäumers noch im Falle des Sonnenblumenöls eine starke Neigung zur Schaumbildung. Nach Abtrennen der Zellen mittels Zentrifugation bei 10.000 g und anschließender Fällung der Rhamnolipide durch Ansäuern auf pH = 4 wird die rhamnolipidhaltige Unterphase abgetrennt und mehrmals mit Wasser bei pH = 4 gewaschen. Das erhaltene Produkt wird durch Zugabe von NaOH wieder auf pH = 7 eingestellt, hierbei gehen die ausgefällten Rhamnolipide wieder in Lösung. Das so erhaltene Produkt weist ungünstige anwendungstechnische Eigenschaften auf. Die über eine SITA Messung analysierte Schaumbildungskinetik und das erzielte Gesamtschaumvolumen sind nicht ausreichend. Im Gegensatz dazu, wird über das hier beanspruchte Verfahren ein Produkt mit hervorragenden Anwendungseigenschaften, insbesondere hervorragenden Schaumeigenschaften erhalten.

Hierbei wird der Extraktionsschritt und die anschließende Aufkonzentrierung wie in den weiter oben beschriebenen Beispielen durchgeführt. Dies kann entweder direkt aus der zellhaltigen Fermentationsbrühe, oder nach Abtrennung der Zellen erfolgen. Wenn die Zellen nicht abgetrennt werden, verläuft die Phasentrennung deutlich langsamer und es bildet sich eine zell- und produkthaltige Zwischenphase, wodurch die Ausbeute reduziert, die Reinheit und die anwendungstechnischen Eigenschaften aber nicht beeinflusst werden. In dem erhaltenen Produkt ist der DOW Corning 1500 Entschäumer bzw. das Sonnenblumenöl über ¹H-NMR nur noch in Spuren nachweisbar. Der Großteil verbleibt in der oberen organischen Phase und kann nach Entfernung des Lösungsmittels optional in einer weiteren Fermentation verwendet werden.

## Patentansprüche

1. Verfahren zur Isolierung von Rhamnolipiden umfassend die Verfahrensschritte
A) Bereitstellen eines mindestens ein Rhamnolipid enthaltenden wässrigen Mediums mit einem pH-Wert kleiner 6,
B) in Kontakt Bringen des Mediums mit mindestens einem organischen Lösungsmittel unter Erhalt eines Mehrphasensystems und Abtrennen der wässrigen Phase,
C) Erhöhung des pH-Wertes auf einen pH-Wert von 6 oder größer unter Erhalt eines mehrphasigen organischen Systems,
D) Abtrennen einer mit Rhamnolipid angereicherten organischen Phase und
E) gegebenenfalls weitere Aufreinigung des Rhamnolipides,
**dadurch gekennzeichnet, dass** das gesamte wässrige Medium in Verfahrensschritt A) zwischen 10 g/L und 300 g/L, insbesondere zwischen 50 g/L und 150 g/L Rhamnolipide enthält.

2. Verfahren gemäß Anspruch 1, **dadurch gekennzeichnet, dass** das wässrige Medium in Verfahrensschritt A) einen pH-Wert von 2 bis 4,5 insbesondere von 3,5 bis 4 aufweist.

3. Verfahren gemäß Anspruch 1 oder 2, **dadurch gekennzeichnet, dass** das organische Lösungsmittel oder die Mischung der organischen Lösungsmittel des Verfahrensschrittes B) Wasser in einer Menge von 0,1 bis 30 Gew.-%, bevorzugt 0,5 bis 7 Gew.-%, zu lösen vermag.

4. Verfahren gemäß mindestens einem der vorherigen Ansprüche, **dadurch gekennzeichnet, dass** das mindestens eine organische Lösungsmittel des Verfahrensschrittes B) ausgewählt ist aus Methylacetat, Ethylacetat, Propylacetat, Isopropylacetat, Butylacetat, Isoamylacetat, Butan-1-ol, Butan-2-ol und Diethylether,.

5. Verfahren gemäß mindestens einem der vorherigen Ansprüche, **dadurch gekennzeichnet, dass** das Volumenverhältnis von wässrigem Medium zu organischem Lösungsmittel oder zu Mischung der organischen Lösungsmittel vor in Kontakt Bringen von 0,2:1 bis 5:1, bevorzugt von 2:1 bis 4:1 und besonders bevorzugt von 2,9:1 bis 3,1:1 beträgt.

6. Verfahren gemäß mindestens einem der vorherigen Ansprüche, **dadurch gekennzeichnet, dass** in Verfahrensschritt C) der pH-Wert auf von 6,1 bis 9, bevorzugt auf von 6,5 bis 8, besonders bevorzugt auf von 6,9 bis 7,1, erhöht wird.

7. Verfahren gemäß mindestens einem der vorherigen Ansprüche, **dadurch gekennzeichnet, dass** in Verfahrensschritt C) der pH-Wert durch eine anorganische Base, insbesondre Erdalkali-, Alkalibase, bevorzugt wässrige, oder Ammoniak erhöht wird, wobei NaOH und KOH besonders bevorzugt sind.

8. Verfahren gemäß mindestens einem der vorherigen Ansprüche, **dadurch gekennzeichnet, dass** die Verfahrensschritte B), C) und D) in einem Temperaturbereich von 10 bis 50 °C, bevorzugt 15 bis 30, besonders bevorzugt 18 bis 25 durchgeführt werden.

9. Verfahren gemäß mindestens einem der vorherigen Ansprüche, **dadurch gekennzeichnet, dass** in Verfahrensschritte E) organisches Lösungsmittel durch mindestens teilweises Verdampfen von dem Rhamnolipid abgetrennt wird.

## Claims

1. Process for the isolation of rhamnolipids comprising the process steps
A) providing an aqueous medium containing at least one rhamnolipid and having a pH of less than 6,
B) bringing the medium into contact with at least one organic solvent to give a multiphase system and separating off the aqueous phase,
C) increasing the pH to a value of 6 or more to give a multiphase organic system,
D) separating off a rhamnolipid-enriched organic phase and
E) optionally further purifying the rhamnolipid,
**characterized in that** the total aqueous medium in process step A) comprises between 10 g/l and 300 g/l, in particular between 50 g/l and 150 g/l, of rhamnolipids.

2. Process according to Claim 1, **characterized in that** the aqueous medium in process step A) has a pH of from 2 to 4.5, in particular from 3.5 to 4.

3. Process according to Claim 1 or 2, **characterized in that** the organic solvent or the mixture of the organic solvents of process step B) is able to dissolve water in an amount of from 0.1 to 30% by weight, preferably 0.5 to 7% by weight.

4. Process according to at least one of the preceding claims, **characterized in that** the at least one organic solvent of process step B) is selected from methyl acetate, ethyl acetate, propyl acetate, isopropyl acetate, butyl acetate, isoamyl acetate, butan-1-ol, butan-2-ol and diethyl ether.

5. Process according to at least one of the preceding claims, **characterized in that** the volume ratio of aqueous medium to organic solvent or to mixture of the organic solvents prior to bringing them into contact is from 0.2:1 to 5:1, preferably from 2:1 to 4:1 and particularly preferably from 2.9:1 to 3.1:1.

6. Process according to at least one of the preceding claims, **characterized in that** in process step C) the pH is increased to from 6.1 to 9, preferably to from 6.5 to 8, particularly preferably to from 6.9 to 7.1.

7. Process according to at least one of the preceding claims, **characterized in that** in process step C) the pH is increased by means of an inorganic base, in particular alkaline earth metal base, alkali base, preferably aqueous, or ammonia, with NaOH and KOH being particularly preferred.

8. Process according to at least one of the preceding claims, **characterized in that** the process steps B), C) and D) are carried out in a temperature range from 10 to 50°C, preferably 15 to 30, particularly preferably 18 to 25.

9. Process according to at least one of the preceding claims, **characterized in that** in process steps E) organic solvent is separated off from the rhamnolipid by means of at least partial evaporation.

## Revendications

1. Procédé d'isolation de rhamnolipides, comprenant les étapes de procédé suivantes :
A) la préparation d'un milieu aqueux contenant au moins un rhamnolipide ayant un pH inférieur à 6,
B) la mise en contact du milieu avec au moins un solvant organique pour obtenir un système multiphasé et la séparation de la phase aqueuse,
C) l'élévation du pH à un pH de 6 ou plus pour obtenir un système organique multiphasé,
D) la séparation d'une phase organique enrichie en rhamnolipide, et
E) éventuellement la purification supplémentaire du rhamnolipide,
**caractérisé en ce que** l'ensemble du milieu aqueux à l'étape de procédé A) contient entre 10 g/l et 300 g/l, notamment entre 50 g/l et 150 g/l, de rhamnolipides.

2. Procédé selon la revendication 1, **caractérisé en ce que** le milieu aqueux à l'étape de procédé A) présente un pH de 2 à 4,5, notamment de 3,5 à 4.

3. Procédé selon la revendication 1 ou 2, **caractérisé en ce que** le solvant organique ou le mélange de solvants organiques de l'étape de procédé B) peut dissoudre de l'eau en une quantité de 0,1 à 30 % en poids, de préférence de 0,5 à 7 % en poids.

4. Procédé selon au moins l'une quelconque des revendications précédentes, **caractérisé en ce que** ledit au moins un solvant organique de l'étape de procédé B) est choisi parmi l'acétate de méthyle, l'acétate d'éthyle, l'acétate de propyle, l'acétate d'isopropyle, l'acétate de butyle, l'acétate d'isoamyle, le butan-1-ol, le butan-2-ol et l'éther diéthylique.

5. Procédé selon au moins l'une quelconque des revendications précédentes, **caractérisé en ce que** le rapport volumique entre le milieu aqueux et le solvant organique ou le mélange de solvants organiques avant la mise en contact est de 0,2:1 à 5:1, de préférence de 2:1 à 4:1 et de manière particulièrement préférée de 2,9:1 à 3,1:1.

6. Procédé selon au moins l'une quelconque des revendications précédentes, **caractérisé en ce qu'**à l'étape de procédé C), le pH est augmenté de 6,1 à 9, de préférence de 6,5 à 8, de manière particulièrement préférée de 6,9 à 7,1.

7. Procédé selon au moins l'une quelconque des revendications précédentes, **caractérisé en ce qu'**à l'étape de procédé C), le pH est augmenté par une base inorganique, notamment une base alcalino-terreuse, alcaline, de préférence aqueuse, ou de l'ammoniac ; NaOH et KOH étant particulièrement préférés.

8. Procédé selon au moins l'une quelconque des revendications précédentes, **caractérisé en ce que** les étapes de procédé B), C) et D) sont réalisées dans une plage de température allant de 10 à 50 °C, de préférence de 15 à 30, de manière particulièrement préférée de 18 à 25.

9. Procédé selon au moins l'une quelconque des revendications précédentes, **caractérisé en ce qu'**à l'étape de procédé E), le solvant organique est séparé du rhamnolipide par évaporation au moins partielle.
